# FASCICULE DE BREVET EUROPEEN

(11) **EP 1 973 425 B1**
(45) Date de publication et mention de la délivrance du brevet: **10.03.2010**
(21) Numéro de dépôt: 07703762.0
(22) Date de dépôt: 10.01.2007
(51) Int. Cl.: A23L 1/29, A23L 1/30, A61K 31/132, A61P 9/06

(54) **UTILISATION NOUVELLE D'UNE COMPOSITION ALIMENTAIRE A USAGE HUMAIN OU VETERINAIRE PAUVRE EN POLYAMINES POUR LA REALISATION D'UN ALIMENT THERAPEUTIQUE**
NEUE VERWENDUNG EINER POLYAMINARMEN NAHRUNGSMITTELZUSAMMENSETZUNG FÜR MENSCH ODER TIER FÜR DIE ZUBEREITUNG EINES THERAPEUTISCHEN NAHRUNGSMITTELPRODUKTS
NOVEL USE OF A POLYAMINE-DEPLETED FOOD COMPOSITION FOR HUMAN OR VETERINARY USE, FOR PREPARING A THERAPEUTIC FOOD PRODUCT

(30) Priorité: 17.01.2006 FR 0600420
(43) Date de publication de la demande: 01.10.2008
(73) Titulaire: Universite de Rennes 1, 35065 Rennes Cedex (FR)
(72) Inventeur: MOULINOUX, Jacques, F-35000 Rennes (FR); ESTEBE, Jean-Pierre, F-35000 Rennes (FR)
(74) Mandataire: Larcher, Dominique
(86) Numéro de dépôt international: PCT/EP2007/050215
(87) Numéro de publication internationale: WO 2007/082822

(56) Documents cités:
- EP-A- 0 270 026
- WO-A-95/00041
- WO-A-2005/020974
- ELIASSEN KA, REISTAD R, RISOEN U, RONNING HF: "Dietary polyamines" FOOD CHEMISTRY, vol. 78, 2002, pages 273-280, XP002398729
- TIL HP, FALKE HE, PRINSEN MK, WILLEMS MI: "Acute and subacute toxicity of tyramine, spermidine, spermine, putrescine and cadaverine in rats" FOOD CHEM. TOXICOL., vol. 35, 1997, pages 337-348, XP002398730

## Description

L'invention concerne les domaines pharmaceutique et vétérinaire.

Plus précisément, l'invention concerne l'utilisation nouvelle de compositions alimentaires pauvres en polyamines pour la réalisation d'aliments, à destination humaine ou animale, susceptibles d'avoir des effets thérapeutiques.

Un objectif de la présente invention est ainsi de proposer un agent thérapeutique susceptible de prévenir ou de traiter les anomalies du rythme cardiaque et ce qu'elle qu'en soit l'étiologie.

Un autre objectif de la présente invention est de proposer un tel agent thérapeutique permettant de lutter contre de telles anomalies apparaissant lors de pathologies déterminées.

Un objectif de la présente invention est aussi de proposer un tel agent thérapeutique permettant de lutter contre de telles anomalies apparaissant suite à un stress (tel que par exemple une opération) ou en cas d'addiction.

Ces objectifs sont atteints grâce à l'invention qui concerne l'utilisation d'une composition alimentaire à usage humain ou vétérinaire présentant moins de 1600 picomoles de polyamines pour la réalisation d'un aliment thérapeutique destiné à prévenir ou à traiter toute anomalie du rythme cardiaque et ce, qu'elle qu'en soit l'étiologie.

On notera que des compositions alimentaires à usage humain sont connues en tant que telles et décrites dans WO-9500041 et WO-2005/020974 au nom de la Demanderesse.

Dans le cadre de WO-9500041, ces compositions sont utilisées comme agent anti-cancéreux (notamment pour le cancer de la prostate), pour stimuler le système immunitaire, pour stimuler l'activité des cellules NK, pour stimuler la production endogène d'interleukine-2, comme agent antalgique et comme agent visant à réduire l'appétit.

Dans le cadre de WO-2005/020974, ces compositions sont utilisées comme agent destinés à lutter contre les syndromes ou pathologies dans lesquels la sous-unité NR2-B du récepteur N-méthyl-D-aspartate est impliquée.

La Demanderesse a maintenant mis en évidence que, de façon surprenante, de telles compositions pouvaient être utilisées pour prévenir ou traiter les anomalies du rythme cardiaque.

De telles compositions alimentaires pourront être administrées par voie buccale, mais aussi par voie entérale par exemple à l'aide d'une sonde.

On rappelle que les polyamines et tout particulièrement la putrescine (I), la spermidine (II) et la spermine (III) sont présentes dans toutes les cellules.

⁺NH₃ - (CH₂)₄ - NH₃⁺ (I)

⁺NH₃ - (CH₂)₃ - NH - (CH₂)₄ - NH₃⁺ (II)

⁺NH3 - (CH₂)₃ - NH - (CH₂)₄ - NH - (CH₂)₃ - NH₃⁺ (III)

Bien que ces molécules aient été longtemps considérées comme dénuées de tout rôle physiologique et ne représentant qu'une étape terminale du catabolisme tissulaire, de nombreux travaux scientifiques ont montré que les polyamines issues de la décarboxylation de l'ornithine étaient en fait des molécules biologiquement actives et capables d'intervenir à différents niveaux importants de la vie de la cellule.

Ces molécules que l'on trouve non seulement à l'intérieur même des cellules mais également à l'état circulant dans les liquides biologiques de l'organisme, tels que le sang sont issues de trois sources principales :
- la prolifération cellulaire physiologique (croissance et/ou renouvellement des cellules constitutives de l'organisme) et tumorale,
- l'alimentation,
- les bactéries intestinales.

Différents travaux ont par ailleurs mis en évidence que, chez l'animal, l'administration conjointe :
- d'une alimentation dépourvue de polyamines,
- d'α-DFMO,
- d'un inhibiteur de la polyamine-oxydase (PAO) supprimant la rétroconversion oxydative de la spermidine et de la spermine en putrescine, et
- de néomycine et de métronidazole,
entraîne une inhibition quasi-totale de la progression tumorale du carcinome pulmonaire de Lewis 3LL (Seiler N. et al, Cancer Research, 1990, n°50, pp. 5077-5083), du glioblastome humain U251 (Moulinoux J-Ph. et al, Anticancer Research, 1991, n°11, pp. 175-180), de l'adénocarcinome de la prostate Dunning MAT-LyLu (Moulinoux J-Ph. et al, Journal of Urology, 1991, n° 146, pp. 1408,1412) et du neuroblastome humain neuro 2a (Quemener et al, "Polyamines in the gastro-intestinal tract", Dowling R.H., Fölsch I.R. et Löser C Ed., Kluwer Academic Publishers Boston, 1992, pp. 375-385).

Il a par ailleurs été également démontré chez l'animal que la déplétion en polyamines pouvait considérablement potentialiser les effets antiprolifératifs des drogues antitumorales conventionnelles (méthotrexate, cyclophosphamide, vindesine) tout en allongeant le temps de survie des animaux et pouvait permettre de réduire les quantités de drogues administrées tout en conservant le même effet antitumoral (Quemener V. et al, "Polyamine deprivation enhances antitumoral efficacy of chemotherapy", Anticancer Research n°12, 1992, pp. 1447-1454).

La présente invention vise donc à couvrir une nouvelle utilisation de telles compositions alimentaires, utilisation non évidente au vu de l'art antérieur, à savoir la prévention ou le traitement des anomalies du rythme cardiaque.

Parmi les syndromes et pathologies dans lesquels on observe une anomalie du rythme cardiaque, on peut citer :
- l'arythmie (tachycardie, bradycardie);
- l'angine de poitrine;
- l'infarctus du myocarde;
- les traumatismes ;
- les opérations ;
- l'anxiété;
- le stress;
- l'addiction, c'est-à-dire, la dépendance vis-à-vis de différentes substances à potentialité toxicomanogène (alcool, tabac, drogues...) et les comportements compulsifs en résultant ;
- l'activité physique ou mentale exagérée.

La présente invention est donc susceptible d'être mise en oeuvre pour traiter ou le cas échéant pour prévenir ces pathologies ou syndromes.

Notamment, les compositions alimentaires en question pourront être administrées à un patient ou à un animal, afin de prévenir toute anomalie du rythme cardiaque pendant une opération, préalablement à celle-ci.

Préférentiellement, la composition utilisée selon la présente invention contient moins d'environ 400 picomoles/g de putrescine, moins d'environ 400 picomoles/g de spermidine, moins d'environ 400 picomoles/g de spermine et moins d'environ 400 picomoles/g de cadavérine.

Préférentiellement, la composition utilisée selon la présente invention contient moins d'environ 400, préférentiellement moins d'environ 200, picomoles/g de polyamines.

Avantageusement, la composition utilisée selon la présente invention contient moins d'environ 100, préférentiellement moins d'environ 50, picomoles/g de putrescine, moins d'environ 100, préférentiellement moins d'environ 50, picomoles/g de spermidine, moins d'environ 100, préférentiellement moins d'environ 50, picomoles/g de spermine et moins d'environ 100, préférentiellement moins d'environ 50, picomoles/g de cadavérine. Une telle composition apporte, journellement, au moins 17 fois moins de putrescine, 40 fois moins de cadavérine, 70 fois moins de spermidine et 220 fois de spermine que l'alimentation naturelle humaine la plus pauvre qui soit en teneur de polyamines, mais qui réponde néanmoins aux besoins nutritionnels humains.

Selon une variante, la composition utilisée selon la présente invention comprend en outre, en pourcentage de poids sec par rapport au poids sec total : 10 à 35% de lipides, 8 à 30% de protéines, 35 à 80% de glucides, jusqu'à 10% d'un mélange constitué de vitamines, de minéraux et d'électrolytes.

Une telle composition pourra être présentée sous forme sèche à dissoudre extemporanément dans un véhicule neutre ou sous forme liquide prête à l'emploi. Dans tous les cas, la composition est présentée sous forme stérile.

Une telle composition est particulièrement bien adaptée à l'homme et constitue un substitutif alimentaire qui permet de carencer les patients de façon efficace en polyamines. Une telle composition permet en effet d'alimenter un patient de façon satisfaisante tout en induisant une carence en polyamines, d'une part en inhibant la synthèse intracellulaire de polyamines et d'autre part en diminuant l'apport de polyamines exogènes.

Une telle composition permet d'inhiber fortement la synthèse endogène des polyamines et diminue de façon très importante l'apport en ces composés puisque les différents ingrédients qui la constituent en sont quasiment dépourvus. Afin de diminuer également les apports en polyamines par les bactéries intestinales, cette composition pourra être administrée concomitamment à une décontamination du tractus gastro-intestinal au moyens d'antibiotique(s) et/ou d'antiparasitaire(s), tels que, par exemple, la néomycine et le métronidazole. On pourra d'ailleurs envisager d'inclure de tels antibiotique(s) et/ou antiparasitaire(s) directement dans ladite composition, sans sortir du cadre de l'invention.

Les nutriments utilisés dans la composition alimentaire selon l'invention possèdent une bonne valeur nutritionnelle même chez les sujets malades.

La quantité d'eau employée pour réaliser la composition utilisée selon la présente invention est déterminée pour que la composition soit plus ou moins liquide et puisse être facilement ingérée par le patient.

Le pourcentage pondéral du mélange constitué de vitamines, de minéraux et d'électrolytes est choisi de façon à répondre aux proportions, connues de l'homme du métier, devant se trouver dans une alimentation équilibrée.

Préférentiellement, la composition utilisée selon la présente invention contient moins de 100 picomoles/g de putrescine, moins de 100 picomoles/g de spermidine, moins de 100 picomoles/g de spermine, moins de 100 picomoles/g de cadavérine.

Une telle composition pourra être administrée conjointement à au moins un inhibiteur de la synthèse intracellulaire des polyamines.

Selon une variante intéressante de l'invention la composition utilisée selon la présente invention est enrichie avec au moins un inhibiteur de la synthèse intracellulaire des polyamines à raison d'au plus 15% en poids par rapport au poids sec total de la composition et, préférentiellement, à raison d'une quantité comprise entre 0,2% et 7% en poids.

Les inhibiteurs de l'ODC utilisables sont choisis en particulier parmi les composés suivants :
Antagonistes du phosphate de pyridoxal
   - L-canatine
   - N-(5'-phosphopyridoxyl)ornithine
Inhibiteurs compétitifs
   - alpha-hydrazino-ornithine
   - acide DL-alpha-hydrazino-delta-aminovalérique
   - alpha-méthylornithine
   - trans-3-déhydro-DL-ornithine
   - 1,4-diamino-trans-2-butène
   - 1,4-diaminobutanone
   - rétinol, rétinoïdes, b-carotènes
   - polyphénols
   - géraniol
   - terpènes
   - flavonoides
   - procyanidines
   - resveratrol
Inhibiteurs diaminés
   - 1,3-diaminopropane
   - 1,3-diamino-2-propanol
   - bis(éthyl)spermine
   - guanidinobutylamine
Inhibiteurs suicides et irréversibles
   - 2-difluorométhylornithine (DFMO)
   - monofluorométhylornithine
   - 2-monofluorométhyldéhydro-ornithine
   - 2-monofluorométhyldéhydro-ornithine méthyl ester
   - 5-hexyne-1,4-diamine
   - trans-hex-2-èn-5-yne-1,4-diamine
   - monofluorométhylputrescine
   - difluorométhylputrescine
   - alpha-allénylputrescine
   - (2R,5R)-6-heptyne-2,5-diamine.

Parmi ces inhibiteurs, les inhibiteurs compétitifs sont particulièrement préférés et notamment l'alpha-méhtylornithine (alpha-MO).

L'alpha-méthylornithine montre de nombreux avantages dans le cadre de l'utilisation proposée ici. En effet, l'alpha-MO présente l'intérêt d'être un composé naturel facilement synthétisable et a une constante d'inhibition élevée.

L'alpha-méthylornithine présente de plus l'avantage d'inhiber la synthèse des polyamines chez Escherischia coli, la bactérie la plus commune peuplant naturellement le tractus intestinal, ce qui n'est notamment pas le cas de 1' -DFMO.

Ainsi l'utilisation d'une composition alimentaire selon l'invention contenant en tant qu'inhibiteur de la synthèse intracellulaire des polyamines, de l'alpha-méthylornithine est-elle susceptible de réduire l'apport exogène de polyamines par les bactéries intestinales sans pour autant avoir recours à une antibiothérapie concomitamment à l'administration de cette composition ou, pour le moins, en permettant de diminuer la dose d'antibiotiques administrée.

Enfin, l'alpha-MO présente l'avantage d'être un simple inhibiteur compétitif de l'ornithine décarboxylase et diminue fortement les risques d'accoutumance de l'organisme par mutation aboutissant à une résistance cellulaire accrue.

Selon une variante, l'utilisation de la composition selon l'invention est enrichie en vitamines, notamment celles apportées, chez l'être humain sain, par les bactéries intestinales. En effet, l'antibiothérapie qui peut accompagner l'administration de ladite composition peut aussi conduire à diminuer l'apport en certaines vitamines. Dans ce cas, il peut s'avérer nécessaire d'enrichir la composition utilisée en ces vitamines afin de ne pas provoquer de carence vitaminique à la suite de l'administration prolongée de ladite composition. Notamment, il pourra s'avérer utile d'enrichir la composition en vitamines ou en dérivés de vitamines. Certains dérivés de la vitamine A (acide rétinoïque) sont en effet des inhibiteurs de l'activité ODC.

Préférentiellement, les glucides de la composition utilisée appartiennent au groupe comprenant les polymères de glucose, les maltodextrines, le saccharose, les amidons modifiés, le glucose monohydraté, le sirop de glucose déshydraté, le monostéarate de glycérol et leurs mélanges. De tels glucides sont en effet digestibles même en cas de pathologie digestive.

Selon une variante de l'invention, les protéines utilisées appartiennent au groupe comprenant les protéines solubles du lait, les protéines de soja, les peptides de sérum, le jaune d'oeuf en poudre, le caséinate de potassium, les peptides non phosphorylés, les peptides de caséine, le caséinate mixte, l'isolat de soja et leurs mélanges.

Préférentiellement, les lipides appartiennent au groupe comprenant l'huile de beurre, l'huile d'arachide, les triglycérides à chaîne moyenne, l'huile de pépins de raisin, l'huile de soja, l'huile d'onagre et leurs mélanges. Avantageusement, lesdits lipides sont constitués par un mélange d'au moins une huile d'origine animale, d'au moins une huile d'origine végétale et de stéarate de glycérol.

Selon une variante de l'invention la composition utilisée selon la présente invention constitue une ration journalière alimentaire d'un être humain et comprend :
- éventuellement ledit inhibiteur de la synthèse intracellulaire des polyamines à raison de moins de 50g et préférentiellement à raison de 1 à 10 g,
- entre 75 g et 500 g de glucides,
- entre 20 g et 185 g de lipides,
- entre 20 g et 225 g de protéines,
- des vitamines, des minéraux et des électrolytes en quantités suffisantes pour répondre aux besoins nutritionnels journaliers d'un être humain.

Les quantités de vitamines, de minéraux et d'électrolytes utilisés sont connues de l'homme du métier et peuvent être facilement trouvées dans la littérature (voir par exemple,"Apports nutritionnels conseillés" Dupin, Abraham et Giachetti deuxième édition 1992, Ed. TEC et DOC Lavoisier)

Une telle composition permet, à elle seule, de répondre aux besoins nutritionnels journaliers d'un patient tout en permettant de réduire la synthèse intracellulaire et l'apport extérieur en polyamines. Elle constitue alors un aliment à part entière.

Bien sûr, il pourra être envisagé d'administrer une telle composition non pas en une seule prise mais en plusieurs prises espacées au cours de la même journée. Chaque ration sera alors définie pondéralement de façon à constituer un sous-multiple d'une ration journalière alimentaire d'un être humain et comprendra:
- éventuellement ledit inhibiteur de la synthèse intracellulaire des polyamines à raison de moins de 50/X g et préférentiellement à raison de 1/X à 10/X g,
- entre 75/X g et 500/X g de glucides,
- entre 20/X g et 185/X g de lipides,
- entre 20/X g et 225/X g de protéines,
- des vitamines, des minéraux et des électrolytes en quantités suffisantes pour répondre partiellement aux besoins nutritionnels journaliers d'un être humain.

X étant un entier compris entre 2 et 8 et correspondant au nombre de rations devant être ingérées par le patient pour satisfaire ses besoins nutritionnels journaliers.

Le nombre de telles rations pourra être choisi de façon à répondre totalement aux besoins alimentaires journaliers du patient ou bien être choisi de façon à ne couvrir qu'une partie des besoins nutritionnels de celui-ci, le reste de ces besoins étant assurés par un alimentation naturelle pauvre en polyamines (jambon et pâtes ou riz par exemple).

Dans ce cas la composition alimentaire sera utilisée en tant que complément alimentaire.

Les inventeurs ont effectué différents travaux, détaillés ci-après, permettant d'établir chez le rat que l'utilisation d'un régime pauvre en polyamines permettait de lutter contre les anomalies du rythme cardiaque.

Vingt rats mâles Srague-Dawley d'un poids moyen de 300 g ont été répartis de façon aléatoire en deux groupes de 10 animaux. Les rats ont été hébergés durant 2 semaines avant réalisation de l'expérimentation dans une animalerie aux nonnes européennes.

Les études ont été réalisées dans le cadre de la convention d'Helsinki, en accord avec les règles édictées par l'International Association for Study of Pain.

Ces deux groupes de 10 rats (5 par cage) ont été alimentés durant 4 jours (96h) avant expérimentation :
- soit par un aliment à teneur normale en polyamines, c'est-à-dire contenant 54 mg.Kg⁻¹ de putrescine, 27 mg.Kg⁻¹ de spermidine, 27 mg mg.Kg⁻¹ de spermine, 37 mg.Kg⁻¹ de cadavérine. Ce groupe est dénommé « groupe témoin » ;
- soit par un aliment à très faible teneur en polyamines contenant moins de 10 µg de polyamines par Kg d'aliment, synthétisé comme précédemment décrit (Kergozien et al., Life Sci. 1996, 58, 2209-15), ceci en accord avec les recommandations de Cheauveau et al. (Arch. Sci. Physiol., 1951, 5, 305-322), et répondant aux besoins nutritionnels journaliers des rongeurs.

Les animaux du groupe témoins et du groupe traités ont ensuite subit une anesthésie générale par inhalation d'halothane à 2% (gaz pour anésthésie générale dilué à raison de 2% avec de l'air).

Les animaux des deux groupes ont ensuite été implantés avec des électrodes externes reliées à un électrocardiographe.

Des électrocardiogrammes (ECG) ont été réalisés durant les 3 premières heures après implantation des électrodes, afin d'établir leur rythme cardiaque basal.

Trois heures après implantation des électrodes cardiaques, chaque animal a reçu une injection d'une solution de 0,2 ml de carragénine à 2% dans du sérum physiologique dans le coussinet plantaire de sa patte arrière droite. La carragénine est une molécule induisant une douleur de type inflammatoire.

Un ECG a été effectué sur chaque rat toutes les minutes, durant 5 minutes, immédiatement après cette injection.

Les animaux ont reçu ensuite une injection d'une solution de 0,5 ml de bupivacaïne à 0,25% dans du sérum physiologique sur le trajet du nerf sciatique. La bupivacaïne est un anesthésique local. Cette injection a été effectuée comme suit : le trajet du nerf sciatique droit a été repéré sur chaque rat *via* l'utilisation d'un stimulateur nerveux externe ((HNS 111 ; Braun Melsungen, Germany). La bupivacaïne a ensuite été injectée sur le trajet dudit nerf au moyen d'une aiguille à insuline (Stimuplex A).

Des tests neurologiques ont été effectués pour vérifier l'anesthésie locale du nerf sciatique. Immédiatement après injection, des ECG ont été réalisés sur chaque rat chaque minute, durant 6 minutes.

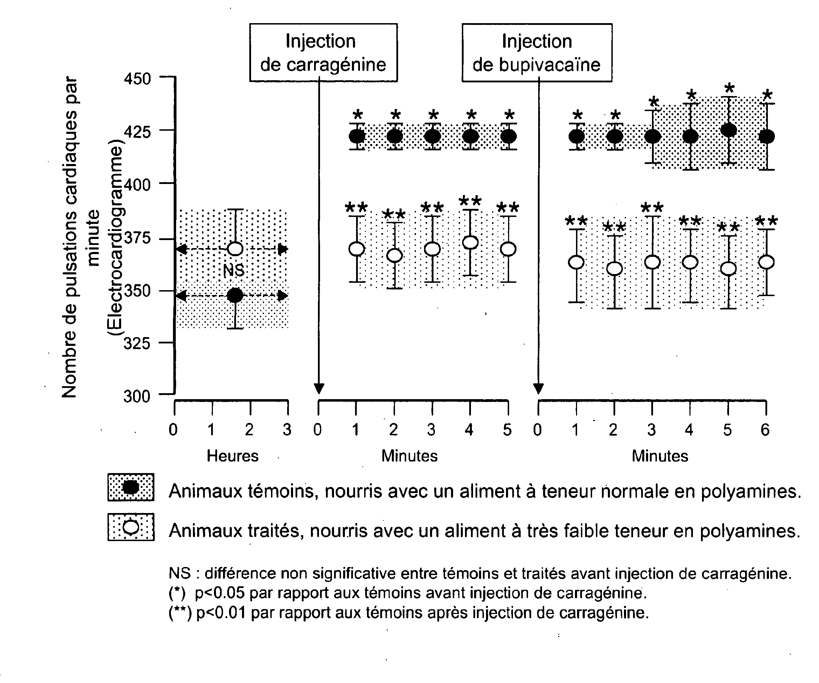

L'essentiel des résultats est reporté dans la Figure 1.

Durant les 3h précédant l'injection de carragénine, et sous anesthésie générale, le rythme cardiaque basal est comparable chez les animaux témoins et chez les animaux traités. Aucune anomalie électrocardiographique (ECG) n'est constatée dans l'un ou l'autre groupe d'animaux.

Toujours sous anesthésie générale, succédant à l'injection de carragénine qui produit à l'état vigile une douleur inflammatoire, le rythme cardiaque des animaux témoins s'accroît de manière très significative et précoce : chez les animaux témoins le rythme cardiaque passe ainsi, moins d'une minute après injection de carragénine, de 350 pulsations par minute à 425; soit une augmentation de 120% de leur rythme cardiaque (tachycardie).

Chez les animaux traités qui ont subi la même injection de carragénine, également sous anesthésie générale, aucune accélération de leur rythme cardiaque n'est enregistrée.

Toujours sous anesthésie générale, l'administration locale de bupivacaïne qui inhibe la transmission de l'influx nerveux du nerf sciatique (nerf moteur et sensitif), ne s'accompagne d'aucune modification significative du rythme cardiaque, tant chez les animaux témoins qui continuent de présenter une tachycardie, que chez les animaux traités qui continuent de posséder un rythme cardiaque normal, comparable à celui constaté avant injection de carragénine.

Chez les animaux traités ayant bénéficié d'une réduction des apports nutritionnels de polyamines durant 4 (quatre) jours avant injection de carragénine, aucune tachycardie n'est constatée, ceci contrairement aux animaux témoins nourris avec un aliment à teneur normale en polyamines.

Or, ces deux groupes d'animaux, témoins et traités, ont subi une injection de carragénine sous anesthésie générale.

Chez des animaux l'injection de carragénine est bien décrite dans la littérature et constitue un modèle expérimental largement utilisé.

De même, la perception d'une douleur s'accompagne en règle générale d'un accroissement du rythme cardiaque. Il en va de manière comparable chez les animaux anesthésiés : il est connu qu'un stimulus douloureux peut générer une tachycardie sous anesthésie générale. C'est le cas lors de l'administration de carragénine.

Dans cette étude, le fait que les animaux témoins sous anesthésie générale, ne bénéficient pas de l'injection locale de bupivacaïne qui inhibe la neuro-transmission de l'information «douleur» de la périphérie (injection de carragénine) vers le système nerveux central via le nerf sciatique, ce que traduit le maintien de la tachycardie, permet d'avancer une origine centrale pour ce qui est du maintien de cette tachycardie.

## Revendications

1. Utilisation d'une composition alimentaire à usage humain ou vétérinaire présentant moins de 1600 picomoles de polyamines pour la réalisation d'un aliment thérapeutique destiné à prévenir ou à traiter toute anomalie du rythme cardiaque et ce, qu'elle qu'en soit l'étiologie.

2. Utilisation selon la revendication 1 d'une composition alimentaire à usage humain ou vétérinaire présentant moins de 1600 picomoles de polyamines destinée à prévenir ou à traiter l'arythmie cardiaque.

3. Utilisation selon la revendication 1 d'une composition alimentaire à usage humain ou vétérinaire présentant moins de 1600 picomoles de polyamines destinée à prévenir ou à traiter l'angine de poitrine.

4. Utilisation selon la revendication 1 d'une composition alimentaire à usage humain ou vétérinaire présentant moins de 1600 picomoles de polyamines destinée à prévenir ou à traiter l'infarctus du myocarde.

5. Utilisation selon la revendication 1 d'une composition alimentaire à usage humain ou vétérinaire présentant moins de 1600 picomoles de polyamines destinée à prévenir ou à traiter toute anomalie du rythme cardiaque provoquée par un traumatisme.

6. Utilisation selon la revendication 1 d'une composition alimentaire à usage humain ou vétérinaire présentant moins de 1600 picomoles de polyamines destinée à prévenir ou à traiter toute anomalie du rythme cardiaque provoquée par une addiction.

7. Utilisation selon la revendication 1 d'une composition alimentaire à usage humain ou vétérinaire présentant moins de 1600 picomoles de polyamines destinée à prévenir ou à traiter toute anomalie du rythme cardiaque provoquée par l'anxiété.

8. Utilisation selon la revendication 1 d'une composition alimentaire à usage humain ou vétérinaire présentant moins de 1600 picomoles de polyamines destinée à prévenir ou à traiter toute anomalie du rythme cardiaque provoquée par une activité mentale et/ou physique exagérée.

9. Utilisation selon l'une quelconque des revendications précédentes **caractérisée en ce que** ladite composition contient moins d'environ 400 picomoles/g de putrescine, moins d'environ 400 picomoles/g de spermidine, moins d'environ 400 picomoles/g de spermine et moins d'environ 400 picomoles/g de cadavérine.

10. Utilisation selon la revendications 9 **caractérisée en ce que** ladite composition contient moins d'environ 400, préférentiellement moins d'environ 200, picomoles/g de polyamines.

11. Utilisation selon la revendication 10 **caractérisée en ce que** ladite composition contient moins d'environ 100, préférentiellement moins d'environ 50, picomoles/g de putrescine, moins d'environ 100, préférentiellement moins d'environ 50, picomoles/g de spermidine, moins d'environ 100, préférentiellement moins d'environ 50, picomoles/g de spermine et moins d'environ 100, préférentiellement moins d'environ 50, picomoles/g de cadavérine.

12. Utilisation selon l'une quelconque des revendications précédentes **caractérisée en ce que** ladite composition contient, en pourcentage de poids sec par rapport au poids sec total : 10 % à 35 % de lipides, 8 % à 30 % de protéines, 35 % à 80 % de glucides, jusqu'à 10 % d'un mélange constitué de vitamines, de minéraux et d'électrolytes.

13. Utilisation selon la revendication 12 **caractérisée en ce que** ladite composition est enrichie avec au moins un inhibiteur de la synthèse intracellulaire des polyamines à raison d'au plus 15 % en poids par rapport au poids sec total de la composition.

14. Utilisation selon la revendication 13 **caractérisée en ce que** ladite composition est enrichie avec ledit inhibiteur à raison de 0,2 % à 7 % en poids par rapport au poids sec total de la composition

15. Utilisation selon la revendication 14 **caractérisée en ce que** ledit inhibiteur de ladite composition est un inhibiteur compétitif de l'ornithine décarboxylase.

16. Utilisation selon la revendication 15 **caractérisée en ce que** ledit inhibiteur compétitif de ladite composition est l'α-méthylomithine.

17. Utilisation selon l'une quelconque des revendications précédentes **caractérisée en ce que** ladite composition contient au moins un antibiotique.

18. Utilisation selon l'une quelconque des revendications précédentes **caractérisée en ce que** ladite composition est enrichie en vitamines.

19. Utilisation selon l'une quelconque des revendications 12 à 18 **caractérisée en ce que** lesdits glucides de la composition appartiennent au groupe comprenant les polymères de glucose, les maltodextrines, le saccharose, les amidons modifiés, le glucose monohydraté, le sirop de glucose déshydraté, le monostéarate de glycérol et leurs mélanges.

20. Utilisation selon l'une quelconque des revendications 12 à 19 **caractérisée en ce que** lesdites protéines de ladite composition appartiennent au groupe comprenant les protéines solubles du lait, les protéines de soja, les peptides de sérum, le jaune d'oeuf en poudre, le caséinate de potassium, les peptides non phosphorylés, les peptides de caséine, le caséinate mixte, l'isolat de soja et leurs mélanges.

21. Utilisation selon l'une quelconque des revendications 12 à 20 **caractérisée en ce que** lesdits lipides de ladite composition appartiennent au groupe comprenant l'huile de beurre, l'huile d'arachide, les triglycérides à chaîne moyenne, l'huile de pépins de raisin, l'huile de soja, l'huile d'onagre et leurs mélanges.

22. Utilisation selon l'une quelconque des revendications 12 à 21 **caractérisée en ce que** lesdits lipides de ladite composition sont constitués par un mélange d'au moins une huile d'origine animale, d'au moins une huile d'origine végétale et de stéarate de glycérol.

23. Utilisation selon l'une quelconque des revendications précédentes **caractérisée en ce que** ladite composition constitue la ration journalière alimentaire d'un être humain et **en ce qu'**elle comprend :
- entre 75 g et 500 g de glucides,
- entre 20 g et 185 g de lipides,
- entre 20 g et 225 g de protéines,
- des vitamines, des minéraux et des électrolytes en quantités suffisantes pour répondre aux besoins nutritionnels journaliers d'un être humain.

24. Utilisation selon l'une quelconque des revendications précédentes **caractérisée en ce que** ladite composition constitue la ration journalière alimentaire d'un être humain et **en ce qu'**elle comprend :
- moins de 50 g et, préférentiellement, entre 1 à 10 g dudit inhibiteur de la synthèse intracellulaire des polyamines,
- entre 75 g et 500 g de glucides,
- entre 20 g et 185 g de lipides,
- entre 20 g et 225 g de protéines,
- des vitamines, des minéraux et des électrolytes en quantités suffisantes pour répondre aux besoins nutritionnels journaliers d'un être humain.

25. Utilisation selon l'une quelconque des revendications précédentes **caractérisée que** ladite composition est un sous-multiple d'une ration journalière alimentaire d'un être humain et en ce qu'elle comprend :
- entre 75/X g et 500/X g de glucides,
- entre 20/X g et 185/X g de lipides,
- entre 20/X g et 225/X g de protéines,
- des vitamines, des minéraux et des électrolytes en quantités suffisantes pour répondre partiellement aux besoins nutritionnels journaliers d'un être humain, et
X étant un entier compris entre 2 et 8 et correspondant au nombre de rations devant être ingérées par le patient pour satisfaire ses besoins nutritionnels journaliers.

26. Utilisation selon l'une quelconque des revendications précédentes **caractérisée en ce que** ladite composition est un sous-multiple d'une ration journalière alimentaire d'un être humain et **en ce qu'**elle comprend :
- moins de 50/X g et, préférentiellement, entre 1/X et 10/X g dudit inhibiteur de la synthèse intracellulaire des polyamines,
- entre 75/X g et 500/X g de glucides,
- entre 20/X g et 185/X g de lipides,
- entre 20/X g et 225/X g de protéines,
- des vitamines, des minéraux et des électrolytes en quantités suffisantes pour répondre partiellement aux besoins nutritionnels journaliers d'un être humain, et
X étant un entier compris entre 2 et 8 et correspondant au nombre de rations devant être ingérées par le patient pour satisfaire ses besoins nutritionnels journaliers.

27. Utilisation selon l'une quelconque des revendications précédentes **caractérisée en ce que** ladite composition se présente sous une forme sèche à dissoudre extemporanément dans un véhicule neutre.

28. Utilisation selon l'une quelconque des revendications précédentes **caractérisée en ce que** ladite composition inclut un véhicule neutre la rendant prête à l'emploi.

## Claims

1. Use of a food composition for human or veterinary use having less than 1600 picomoles of polyamines for preparing a therapeutic food product intended to prevent or treat any heart rate anomaly, irrespective of the etiology.

2. Use according to claim 1 of a food composition for human or veterinary use which having than 1600 picomoles of polyamines intended to prevent or treat cardiac arrhythmia.

3. Use according to claim 1 of a food composition for human or veterinary use having less than 1600 picomoles of polyamines intended to prevent or treat angina pectoris.

4. Use according to claim 1 of a food composition for human or veterinary use having less than 1600 picomoles of polyamines intended to prevent or treat myocardial infarction.

5. Use according to claim 1 of a food composition for human or veterinary use having less than 1600 picomoles of polyamines intended to prevent or treat any heart rate anomaly induced by trauma.

6. Use according to claim 1 of a food composition for human or veterinary use having less than 1600 picomoles of polyamines intended to prevent or treat any heart rate anomaly induced by addiction.

7. Use according to claim 1 of a food composition for human or veterinary use having less than 1600 picomoles of polyamines intended to prevent or treat any heart rate anomaly induced by anxiety.

8. Use according to claim 1 of a food composition for human or veterinary use having less than 1600 picomoles of polyamines intended to prevent or treat any heart rate anomaly induced by excessive mental and/or physical activity.

9. Use according to the any of the above claims **characterised in that** said composition contains less than approximately 400 picomoles/g of putrescine, less than approximately 400 picomoles/g of spermidine, less than approximately 400 picomoles/g of spermine and less than approximately 400 picomoles/g of cadaverine.

10. Use according to claim 9 **characterised in that** said composition contains less than approximately 400, preferentially less than approximately 200, picomoles/g ofpolyamines.

11. Use according to claim 10 **characterised in that** said composition contains less than approximately 100, preferentially less than approximately 50, picomoles/g of putrescine, less than approximately 100, preferentially less than approximately 50, picomoles/g of spermidine, less than approximately 100, preferentially less than approximately 50, picomoles/g of spermine and less than approximately 100, preferentially less than approximately 50, picomoles/g of cadaverine.

12. Use according to any of the above claims **characterised in that** said composition contains, as a percentage of dry weight with respect to the total dry weight: 10% to 35% lipids, 8% to 30% protein, 35% to 80% carbohydrates, up to 10% of a mixture consisting of vitamins, minerals and electrolytes.

13. Use according to claim 12 **characterised in that** said composition is enriched with at least one inhibitor of the intracellular polyamine synthesis at a rate of not more than 15% by weight with respect to the total dry weight of the composition.

14. Use according to claim 13 **characterised in that** said composition is enriched with said inhibitor at a rate of between 0.2% and 7% by weight with respect to the total dry weight of the composition.

15. Use according to claim 14 **characterised in that** said inhibitor of said composition is a competitive inhibitor of ornithine decarboxylase.

16. Use according to claim 15 **characterised in that** said competitive inhibitor of said composition is α-methylomithine.

17. Use according to any of the above claims **characterised in that** said composition contains at least one antibiotic.

18. Use according to any of the above claims **characterised in that** said composition is enriched with vitamins.

19. Use according to any of claims 12 to 18 **characterised in that** said carbohydrates of the composition belong to the group comprising glucose polymers, maltodextrins, saccharose, modified starches, glucose monohydrate, dehydrated glucose syrup, glycerol monostearate and mixtures thereof.

20. Use according to any of claims 12 to 19 **characterised in that** said proteins of the said composition belong to the group comprising soluble milk proteins, soya proteins, serum peptides, powdered egg yolk, potassium caseinate, non-phosphorylated peptides, casein peptides, mixed caseinate, soya isolate and mixtures thereof.

21. Use according to any of claims 12 to 20 **characterised in that** said lipids of the said composition belong to the group comprising butter oil, peanut oil, medium-chain triglycerides, grape seed oil, soya oil, oil of evening primrose and mixtures thereof.

22. Use according to any of claims 12 to 21 **characterised in that** said lipids of said composition consist of a mixture of at least one oil of animal origin, at least one oil of vegetable origin and glycerol stearate.

23. Use according to any of the above claims **characterised in that** said composition represents a daily nutritional intake for a human and **in that** it comprises:
- between 75 g and 500 g of carbohydrates,
- between 20 g and 185 g of lipids,
- between 20 g and 225 g of protein,
- vitamins, minerals and electrolytes in sufficient quantities to meet a human's daily nutritional requirements.

24. Use according to any of the above claims **characterised in that** said composition represents a daily nutritional intake for a human and **in that** it comprises:
- less than 50 g and, preferentially, between 1 and 10 g of said inhibitor of the intracellular polyamine synthesis,
- between 75 g and 500 g of carbohydrates,
- between 20 g and 185 g of lipids,
- between 20 g and 225 g of protein,
- vitamins, minerals and electrolytes in sufficient quantities to meet a human's daily nutritional requirements.

25. Use according to any of the above claims **characterised in that** said composition is a divisor of a human's daily nutritional intake and **in that** it comprises:
- between 75/X g and 500/X g of carbohydrates,
- between 20/X g and 185/X g of fat,
- between 20/X g and 225/X g of protein,
- vitamins, minerals and electrolytes in sufficient quantities to partially meet a human's daily nutritional requirements, and
X being an integer between 2 and 8 and corresponding to the number of intakes to be ingested by the patient in order to meet his/her daily nutritional requirements.

26. Use according to any of the above claims **characterised in that** said composition is a divisor of a human's daily nutritional intake and **in that** it comprises:
- less than 50/X g and, preferentially, between 1/X and 10/X g of said inhibitor of the intracellular polyamine synthesis,
- between 75/X g and 500/X g of carbohydrates,
- between 20/X g and 185/X g of lipids,
- between 20/X g and 225/X g of protein,
- vitamins, minerals and electrolytes in sufficient quantities to partially meet a human's daily nutritional requirements, and
X being an integer between 2 and 8 and corresponding to the number of intakes to be ingested by the patient in order to meet his/her daily nutritional requirements.

27. Use according to any of the above claims **characterised in that** said composition is presented in a dry form to be dissolved extemporaneously in a neutral vehicle.

28. Use according to any of the above claims **characterised in that** said composition includes a neutral vehicle rendering it ready for use.

## Patentansprüche

1. Verwendung einer Nahrungsmittelzusammensetzung für den menschlichen oder tierärztlichen Einsatz, mit einem Gehalt von weniger als 1600 Picomol an Polyaminen, zur Herstellung eines therapeutischen Nahrungsmittels zum Vorbeugen oder Behandeln aller Herzrhythmusanomalien, unabhängig von deren Ätiologie .

2. Verwendung einer Nahrungsmittelzusammensetzung für den menschlichen oder tierärztlichen Einsatz mit einem Gehalt von weniger als 1600 Picomol an Polyaminen nach Anspruch 1 zum Vorbeugen oder Behandeln von Herzrhythmusstörungen.

3. Verwendung einer Nahrungsmittelzusammensetzung für den menschlichen oder tierärztlichen Einsatz mit einem Gehalt von weniger als 1600 Picomol an Polyaminen nach Anspruch 1 zum Vorbeugen oder Behandeln von Angina Pectoris.

4. Verwendung einer Nahrungsmittelzusammensetzung für den menschlichen oder tierärztlichen Einsatz mit einem Gehalt von weniger als 1600 Picomol an Polyaminen nach Anspruch 1 zum Vorbeugen oder Behandeln von Herzinfarkten.

5. Verwendung einer Nahrungsmittelzusammensetzung für den menschlichen oder tierärztlichen Einsatz mit einem Gehalt von weniger als 1600 Picomol an Polyaminen nach Anspruch 1 zum Vorbeugen oder Behandeln aller durch Traumata verursachten Herzrhythmusanomalien.

6. Verwendung einer Nahrungsmittelzusammensetzung für den menschlichen oder tierärztlichen Einsatz mit einem Gehalt von weniger als 1600 Picomol an Polyaminen nach Anspruch 1 zum Vorbeugen oder Behandeln aller durch Sucht verursachten Herzrhythmusanomalien.

7. Verwendung einer Nahrungsmittelzusammensetzung für den menschlichen oder tierärztlichen Einsatz mit einem Gehalt von weniger als 1600 Picomol an Polyaminen nach Anspruch 1 zum Vorbeugen oder Behandeln aller durch Angst verursachten Herzrhythmusanomalien.

8. Verwendung einer Nahrungsmittelzusammensetzung für den menschlichen oder tierärztlichen Einsatz mit einem Gehalt von weniger als 1600 Picomol an Polyaminen nach Anspruch 1 zum Vorbeugen oder Behandeln aller durch übermäßige geistige und/oder körperliche Aktivitäten verursachten Herzrhythmusanomalien.

9. Verwendung nach einem der vorhergehenden Ansprüche, **dadurch gekennzeichnet, dass** die besagte Zusammensetzung weniger als ca. 400 Picomol/g Putrescin, weniger als ca. 400 Picomol/g Spermidin, weniger als ca. 400 Picomol/g Spermin und weniger als ca. 400 Picomol/g Kadaverin enthält.

10. Verwendung nach Anspruch 9, **dadurch gekennzeichnet, dass** die besagte Zusammensetzung weniger als ca. 400, bevorzugterweise weniger als ca. 200 Picomol/g Polyamine enthält.

11. Verwendung nach Anspruch 10, **dadurch gekennzeichnet, dass** die besagte Zusammensetzung weniger als ca. 100, bevorzugterweise weniger als ca. 50 Picomol/g Putrescin, weniger als ca. 100, bevorzugterweise weniger als ca. 50 Picomol/g Spermidin, weniger als ca. 100, bevorzugterweise weniger als ca. 50 Picomol/g Spermin und weniger als ca. 100, bevorzugterweise weniger als ca. 50 Picomol/g Kadaverin enthält.

12. Verwendung nach einem der vorhergehenden Ansprüche, **dadurch gekennzeichnet, dass** die besagte Zusammensetzung in Prozent des Trockengewichts im Verhältnis zum gesamten Trockengewicht folgendes enthält: 10 % bis 35 % Lipide, 8 % bis 30 % Proteine, 35% bis 80 % Kohlenhydrate und bis zu 10 % einer Mischung, die aus Vitaminen, Mineralien und Elektrolyten besteht.

13. Verwendung nach Anspruch 12, **dadurch gekennzeichnet, dass** die besagte Zusammensetzung mit mindestens einem intrazellulären Inhibitor der Polyaminsynthese in einem Maße von nicht mehr als 15 Gewichtsprozent im Verhältnis zum gesamten Trockengewicht der Zusammensetzung angereichert ist.

14. Verwendung nach Anspruch 13, **dadurch gekennzeichnet, dass** die besagte Zusammensetzung mit dem besagten Inhibitor in einem Maße von nicht mehr als 0,2 bis 7 Gewichtsprozent im Verhältnis zum gesamten Trockengewicht der Zusammensetzung angereichert ist.

15. Verwendung nach Anspruch 14, **dadurch gekennzeichnet, dass** der besagte Inhibitor der besagten Zusammensetzung ein kompetitiver Inhibitor der Ornithin Decarboxylase ist.

16. Verwendung nach Anspruch 15, **dadurch gekennzeichnet, dass** es sich beim besagten kompetitiven Inhibitor der besagten Zusammensetzung um α-Methylornithin handelt.

17. Verwendung nach einem der vorhergehenden Ansprüche, **dadurch gekennzeichnet, dass** die besagte Zusammensetzung mindestens ein Antibiotikum enthält.

18. Verwendung nach einem der vorhergehenden Ansprüche, **dadurch gekennzeichnet, dass** die besagte Zusammensetzung mit Vitaminen angereichert ist.

19. Verwendung nach einem der Ansprüche 12 bis 18, **dadurch gekennzeichnet, dass** die besagten Kohlenhydrate der Zusammensetzung zu der Gruppe gehören, welche Glucosepolymere, Maltodextrine, Saccharosen, modifizierte Stärken, Glucosemonohydrate, dehydrierten Glucosesirup, Glyzerinmonostearat und deren Mischungen umfasst.

20. Verwendung nach einem der Ansprüche 12 bis 19, **dadurch gekennzeichnet, dass** die besagten Proteine der besagten Zusammensetzung zu der Gruppe gehören, welche lösliche Milchproteine, Sojaproteine, Serumpeptide, pulverförmiges Eigelb, Kaliumkaseinate, nicht phosphorylierte Peptide, Kaseinpeptide, gemischtes Kaseinat sowie Sojaisolat und deren Mischungen umfasst.

21. Verwendung nach einem der Ansprüche 12 bis 20, **dadurch gekennzeichnet, dass** die besagten Lipide der besagten Zusammensetzung zu der Gruppe gehören, welche Butterschmalz, Erdnussöl, Triglyzeride mittlerer Kettenlänge, Traubenkernöl, Sojaöl, Nachtkerzenöl und deren Mischungen umfasst.

22. Verwendung nach einem der Ansprüche 12 bis 21, **dadurch gekennzeichnet, dass** die besagten Lipide der besagten Zusammensetzung aus einem Gemisch aus mindestens einem tierischen Öl, mindestens einem Pflanzenöl und Glycerolstearat bestehen.

23. Verwendung nach einem der vorhergehenden Ansprüche, **dadurch gekennzeichnet, dass** die besagte Zusammensetzung die tägliche Nahrungseinnahme eines Menschen darstellt und folgendes enthält:
- 75 g bis 500 g Kohlenhydrate,
- 20 g bis 185 g Lipide,
- 20 g bis 225 g Protein,
- Vitamine, Mineralstoffe und Elektrolyte in ausreichender Menge um den täglichen Ernährungsbedarf eines Menschen zu decken.

24. Verwendung nach einem der vorhergehenden Ansprüche, **dadurch gekennzeichnet, dass** die besagte Zusammensetzung die tägliche Nahrungseinnahme eines Menschen darstellt und folgendes enthält:
- weniger als 50 g, bevorzugterweise zwischen 1 und 10 g des besagten intrazellulären Inhibitors der Polyaminsynthese,
- 75 g bis 500 g Kohlenhydrate,
- 20 g bis 185 g Lipide,
- 20 g bis 225 g Protein,
- Vitamine, Mineralstoffe und Elektrolyte in ausreichender Menge um den täglichen Ernährungsbedarf eines Menschen zu decken.

25. Verwendung nach einem der vorhergehenden Ansprüche, **dadurch gekennzeichnet, dass** die besagte Zusammensetzung ein Teil der täglichen Nahrungsmittelration eines Menschen ist, welcher folgendes enthält:
- 75/X g bis 500/X g Kohlenhydrate,
- 20/X g bis 185/X g Lipide,
- 20/X g bis 225/X g Protein,
- Vitamine, Mineralstoffe und Elektrolyte in ausreichender Menge, um einen Teil des täglichen Ernährungsbedarfs eines Menschen zu decken, und
wobei X eine Ganze Zahl zwischen 2 und 8 ist und der Anzahl von Portionen entspricht, die der Patient zu sich nehmen muss, um seinen täglichen Nahrungsbedarf zu decken.

26. Verwendung nach einem der vorhergehenden Ansprüche, **dadurch gekennzeichnet, dass** die besagte Zusammensetzung ein Teil der täglichen Nahrungsmittelration eines Menschen ist, welcher folgendes enthält:
- weniger als 50/X g, bevorzugterweise zwischen 1/X und 10/X g des besagten intrazellulären Inhibitors der Polyaminsynthese,
- 75/X g bis 500/X g Kohlenhydrate,
- 20/X g bis 185/X g Lipide,
- 20/X g bis 225/X g Protein,
- Vitamine, Mineralstoffe und Elektrolyte in ausreichender Menge, um einen Teil des täglichen Ernährungsbedarfs eines Menschen zu decken, und
wobei X eine Ganze Zahl zwischen 2 und 8 ist, und der Anzahl von Portionen entspricht, die der Patient zu sich nehmen muss, um seinen täglichen Nahrungsbedarf zu decken.

27. Verwendung nach einem der vorhergehenden Ansprüche, **dadurch gekennzeichnet, dass** die besagte Zusammensetzung in trockener Form vorliegt, um bei Bedarf in einer neutralen Trägersubstanz gelöst zu werden.

28. Verwendung nach einem der vorhergehenden Ansprüche, **dadurch gekennzeichnet, dass** die besagte Zusammensetzung eine neutrale Trägersubstanz enthält, welche sie sofort einsatzbereit macht.
